# FASCICULE DE BREVET EUROPEEN

(11) **EP 1 800 678 B1**
(45) Date de publication et mention de la délivrance du brevet: **11.05.2011**
(21) Numéro de dépôt: 06291993.1
(22) Date de dépôt: 20.12.2006
(51) Int. Cl.: A61K 31/4045, A61K 31/55, A61K 45/06, A61P 9/12

(54) **Association d'un inhibiteur du courant if sinusal et d'un inhibiteur de l'enzyme de conversion de l'angiotensine**
Kombination eines Inhibitoren des If-Herzstromes am Sinusknoten und eines Inhibitoren des Angiotensin-umwandelnden Enzyms
Combination of an inhibitor of the If current of the sinus node and an inhibitor of the angiotensin converting enzyme

(30) Priorité: 21.12.2005 FR 0513006
(43) Date de publication de la demande: 27.06.2007
(60) Demande divisionnaire: 10011132.7
(73) Titulaire: Les Laboratoires Servier, 92415 Courbevoie Cédex (FR)
(72) Inventeur: Benatar, Vidal, 92210 Saint-Cloud (FR); Lerebours-Pigeonniere, Guy, 92300 Levallois-Perret (FR)
(74) Mandataire: Bestel, Delphine

(56) Documents cités:
- EP-A- 0 471 388
- EP-A- 1 362 590
- WO-A-01/78699
- WO-A-02/100328
- MATOS J P ET AL: "Effects of perindopril, irbesartan, and perindopril plus irbesartan on blood pressure, renal hemodynamics, proteinuria, renin angiotesin-aldosterone system, and TGF-B1, in type 2 diabetes with nephropathy" AMERICAN JOURNAL OF HYPERTENSION, ELSEVIER, vol. 17, no. 5, mai 2004 (2004-05), page S91, XP004575638 ISSN: 0895-7061
- ALBALADEJO PIERRE ET AL: "Selective reduction of heart rate by ivabradine: effect on the visco-elastic arterial properties in rats" JOURNAL OF HYPERTENSION, vol. 22, no. 9, septembre 2004 (2004-09), pages 1739-1745, XP009070198 ISSN: 0263-6352
- ALBALADEJO P ET AL: "Effect of chronic heart rate reduction with ivabradine on carotid and aortic structure and function in normotensive and hypertensive rats." JOURNAL OF VASCULAR RESEARCH, vol. 40, no. 4, juillet 2003 (2003-07), - août 2003 (2003-08) pages 320-328, XP009070211 ISSN: 1018-1172
- BORER JEFFREY S ET AL: "Characterization of the heart rate-lowering action of ivabradine, a selective I(f) current inhibitor.", AMERICAN JOURNAL OF THERAPEUTICS 2008 SEP-OCT LNKD- PUBMED:18806523, vol. 15, no. 5, September 2008 (2008-09), pages 461-473, ISSN: 1536-3686
- LERMAN LILACH O ET AL: "Animal models of hypertension: An overview", JOURNAL OF LABORATORY AND CLINICAL MEDICINE, vol. 146, no. 3, September 2005 (2005-09), pages 160-173, ISSN: 0022-2143
- VELKOSKA E ET AL: "Beneficial effects of ivabradine on cardiac remodelling in the diabetic hypertensive heart are mediated through modulation of connective tissue growth factor", EUROPEAN HEART JOURNAL, vol. 30, no. 1, P3382, 1 September 2009 (2009-09-01), pages 575-576,

## Description

La présente invention concerne une nouvelle association d'un inhibiteur sélectif et spécifique du courant I_{f} sinusal et d'un agent inhibiteur de l'enzyme de conversion de l'angiotensine.
Plus particulièrement, la présente invention concerne une nouvelle association d'un inhibiteur sélectif et spécifique du courant I_{f} sinusal qui est l'ivabradine ou 3-{3-[{[(7S) - 3,4-diméthoxybicyclo[4.2.0]octa-1,3,5-trién-7-yl]méthyl}(méthyl)amino]propyl}-7,8-diméthoxy-1,3,4,5-tétrahydro-2*H*-3-benzazépin-2-one de formule (I) : 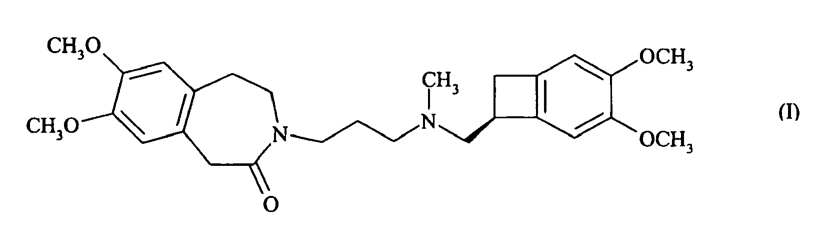 ainsi que ses hydrates, formes cristallines et sels d'addition à un acide pharmaceutiquement acceptable, et d'un agent inhibiteur de l'enzyme de conversion de l'angiotensine.

Les inhibiteurs sélectifs et spécifiques du courant I_{f} sinusal et plus particulièrement l'ivabradine, ainsi que ses hydrates, formes cristallines et ses sels d'addition à un acide pharmaceutiquement acceptable, et plus particulièrement son chlorhydrate, possèdent des propriétés pharmacologiques et thérapeutiques très intéressantes, notamment des propriétés chronotropes négatives (réduction de la fréquence cardiaque), qui rendent ces composés utiles dans le traitement, la prévention et l'amélioration du pronostic de différentes maladies cardio-vasculaires liées à l'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque chronique.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

L'article de Albaladejo P et al (Journal of Hypertension, September 2004, volume 22, number 9, pages 1739-1745) décrit l'effet antihypertenseur de l'ivabradine seule administrée par voie intraveineuse sous forme de 4 doses consécutives toutes les 10 minutes chez des rats SHR (Spontaneously Hypertensive Rats).

La demanderesse a présentement découvert que, de façon surprenante, l'inhibiteur sélectif et spécifique du courant I_{f} sinusal l'ivabradine ou 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2*H*-3-benzazépin-2-one, utilisé en association avec un agent inhibiteur de l'enzyme de conversion de l'angiotensine, possédait des propriétés intéressantes permettant l'utilisation de cette association dans le traitement de l'hypertension artérielle.

L'hypertension artérielle est une maladie silencieuse, mais insidieuse : si, la plupart du temps, elle ne s'accompagne d'aucun trouble immédiat, elle se manifeste - quand elle n'est pas traitée - au bout de 10 à 20 ans, par la survenue d'un grave accident vasculaire, cardiaque ou cérébral. Au-delà d'un paramètre biologique défini par le dépassement d'une norme, fixée par des experts, l'hypertension artérielle constitue un facteur de risque majeur des maladies cardiovasculaires - qui représentent la première cause de mortalité du dernier tiers de la vie humaine, dans les pays industrialisés.
Cette "bombe à retardement" sanitaire, pose donc un très vaste problème de santé publique, compte tenu de sa fréquence élevée parmi la population générale. Sur le plan thérapeutique, les mesures hygieno-diététiques sont toujours nécessaires. Elles permettent d'éviter parfois, mais le plus souvent simplement de retarder la mise en route d'un traitement médicamenteux. L'arsenal médicamenteux est large mais reste souvent insuffisant pour obtenir un contrôle satisfaisant de la pression artérielle.
On sait que le niveau de pression artérielle (systolique ou diastolique) est un déterminant très important du risque de survenue d'un accident vasculaire cérébral ou d'un infarctus du myocarde. De très nombreux essais cliniques ont démontré que la réduction de la pression artérielle diminuait de façon très significative le risque d'accidents cérébraux et cardiaques chez les hypertendus.
Il est connu que la réponse individuelle à un traitement antihypertenseur est variable et difficilement prévisible. L'obtention d'un contrôle optimal de la pression artérielle nécessite le recours à une plurithérapie (avec des médicaments de mécanisme d'action différents) chez la plupart des patients hypertendus.
On recherche toujours des molécules actives sur les formes d'hypertension qui résistent aux traitements actuels. Par ailleurs, on recherche également des médicaments à plus longue durée d'action, ou ayant encore moins d'effets secondaires cliniques ou biologiques.
Ainsi, l'élaboration de nouveaux traitements alternatifs est toujours d'actualité et reste une nécessité.

Une classe thérapeutique largement utilisée dans le traitement de l'hypertension artérielle est constituée par les inhibiteurs de l'enzyme de conversion de l'angiotensine (IEC).
Les inhibiteurs de l'enzyme de conversion sont une des classes thérapeutiques majeures dans le traitement de l'hypertension artérielle. Ils agissent principalement par l'inhibition de la synthèse de l'angiotensine II et par un blocage de la dégradation de la bradykinine.
Leurs effets hémodynamiques consistent essentiellement en la diminution des résistances périphériques totales par vasodilatation artériolaire, ce qui entraîne une baisse de la pression artérielle sans stimulation sympathique ni rétention hydrosodée. Ils sont efficaces dans tous les types d'hypertension artérielle. Ils ont montré qu'au delà de la baisse de la pression artérielle ils amélioraient la morbidité (infarctus du myocarde, accidents vasculaires cérébraux) et la mortalité cardiovasculaire des hypertendus, des diabétiques, des malades avec une maladie coronaire préexistante.
Ils sont en général très bien tolérés à part l'apparition chez certains patients d'une toux sèche, réversible à l'arrêt du traitement.

La demanderesse a présentement découvert que, de façon surprenante, l'inhibiteur sélectif et spécifique du courant I_{f} sinusal ivabradine était capable de potentialiser les effets des agents inhibiteurs de l'enzyme de conversion de l'angiotensine. Cet effet, non prévisible du fait même de la classe thérapeutique des inhibiteurs sélectifs et spécifiques du courant I_{f} sinusal, permet d'envisager l'utilisation de l'association selon l'invention dans le traitement de l'hypertension artérielle, et plus particulièrement, cette potentialisation des effets permettra d'utiliser l'association selon l'invention dans le traitement de patients non contrôlés par les associations thérapeutiques classiques.

Les IEC utilisables dans l'association selon l'invention sont, à titre non limitatif : le Perindopril, le Captopril, l'Enalapril, le Lisinopril, le Delapril, le Fosinopril, le Quinapril, le Ramipril, le Spirapril, l'Imidapril, le Trandolapril, le Benazepril, le Cilazapril et le Temocapril, ainsi que leurs hydrates, formes cristallines et sels d'addition à un acide ou à une base pharmaceutiquement acceptable.

Les IEC préférés sont le Perindopril, le Captopril, l'Enalapril, le Ramipril, le Lisinopril, le Benazapril, le Quinapril et le Delapril ainsi que leurs hydrates, formes cristallines et sels d'addition à un acide ou à une base pharmaceutiquement acceptable, et plus spécialement le Perindopril ou un de ses hydrates, formes cristallines et sels d'addition à un acide ou à une base pharmaceutiquement acceptable, et plus particulièrement ses sels de *tert*-butylamine ou d'arginine.

l'inhibiteur sélectif et spécifique du courant I_{f} sinusal est l'ivabradine ainsi que ses hydrates, formes cristallines et sels d'addition à un acide pharmaceutiquement acceptable.

Plus particulièrement, l'invention concerne l'association de l'ivabradine ou un de ses hydrates, formes cristallines, et sels d'addition à un acide pharmaceutiquement acceptable et plus particulièrement son chlorhydrate, et d'un agent inhibiteur de l'enzyme de conversion de l'angiotensine ou un de ses hydrates, formes cristallines, et sels d'addition à un acide pharmaceutiquement acceptable.

Plus particulièrement, l'invention concerne l'association entre l'ivabradine ou un de ses hydrates, formes cristallines, et sels d'addition à un acide pharmaceutiquement acceptable et plus particulièrement son chlorhydrate, et le perindopril ou un de ses hydrates, formes cristallines et sels d'addition à une base pharmaceutiquement acceptable, et plus particulièrement ses sels d'arginine ou de *tert*-butylamine.

Plus particulièrement, l'invention concerne les compositions pharmaceutiques contenant l'association d'un inhibiteur sélectif et spécifique du courant I_{f} sinusal qui est l'ivabradine ainsi que ses hydrates, formes cristallines et sels d'addition à un acide pharmaceutiquement acceptable et plus particulièrement son chlorhydrate, et d'un agent inhibiteur de l'enzyme de conversion de l'angiotensine ou un de ses hydrates, formes cristallines, et sels d'addition à un acide pharmaceutiquement acceptable, en combinaison avec un ou plusieurs excipients pharmaceutiquement acceptable.

Préférentiellement, l'invention concerne les compositions pharmaceutiques contenant l'association d'un inhibiteur sélectif et spécifique du courant I_{f} sinusal qui est l'ivabradine ainsi que ses hydrates, formes cristallines et sels d'addition à un acide pharmaceutiquement acceptable et plus particulièrement son chlorhydrate, et d'un agent inhibiteur de l'enzyme de conversion de l'angiotensine qui est le périndopril ou un de ses hydrates, formes cristallines, et sels d'addition à une base pharmaceutiquement acceptable et plus particulièrement ses sels d'arginine ou de tert-butylamine, en combinaison avec un ou plusieurs excipients pharmaceutiquement acceptable.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc... ainsi que les compositions pharmaceutiques avec libération programmée, retardée, prolongée ou différée.

Outre l'inhibiteur sélectif et spécifique du courant I_{f} sinusal et le composé inhibiteur de l'enzyme de conversion de l'angiotensine, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne de 1 à 500 mg d'ivabradine par 24 heures et plus préférentiellement 10 à 15 mg par jour et de manière encore préférée de 5 à 15mg par jour. La dose de l'agent inhibiteur de l'enzyme de conversion de l'angiotensine pourra être moindre que celle utilisée lorsqu'il est administré seul.

Les exemples suivants illustrent l'invention et ne la limitent en aucune façon.

### Compositions pharmaceutiques :

| Formule de préparation pour 1000 comprimés dosés à 7,5 mg d'ivabradine et 2 mg de périndopril : | |
|---|---|
| Ivabradine, chlorhydrate | 7,5 g |
| Périndopril, *tert*-butylamine | 2 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

D'autres exemples de compositions pharmaceutiques selon l'invention sont donnés ci-dessous, à titre non limitatif :

### Exemple 1

| Constituants | Quantité (mg) |
|---|---|
| ivabradine | 10 |
| périndopril, sel de tert-butylamine | 4 |

### Exemple 2

| Constituants | Quantité (mg) |
|---|---|
| ivabradine | 10 |
| périndopril, sel de tert-butylamine | 8 |

### Exemple 3

| Constituants | Quantité (mg) |
|---|---|
| ivabradine | 15 |
| périndopril, sel de tert-butylamine | 4 |

### Exemple 4

| Constituants | Quantité (mg) |
|---|---|
| ivabradine | 15 |
| périndopril, sel de tert-butylamine | 8 |

### Exemple 5

| Constituants | Quantité (mg) |
|---|---|
| ivabradine | 10 |
| périndopril, sel d'arginine | 5 |

### Exemple 6

| Constituants | Quantité (mg) |
|---|---|
| ivabradine | 10 |
| périndopril, sel d'arginine | 10 |

### Exemple 7

| Constituants | Quantité (mg) |
|---|---|
| ivabradine | 15 |
| périndopril, sel d'arginine | 5 |

### Exemple 8

| Constituants | Quantité (mg) |
|---|---|
| ivabradine | 15 |
| périndopril, sel d'arginine | 10 |

La posologie pour les compositions pharmaceutiques décrites supra consiste en une administration per os d'un comprimé par 24 heures.

Dans les populations à risque correspondant à des patients âgés de plus de 75 ans, la dose critique initiale administrée par voie orale est de 5mg d'ivabradine et 2mg de périndopril, sel de tert-butylamine ou de 5mg d'ivabradine et 2.5mg de périndopril, sel d'arginine par 24 heures sous la forme d'un comprimé.

### Etude clinique n°1 :

Une étude clinique a été menée chez 8 patients présentant une hypertension artérielle qui étaient déjà traités avec un inhibiteur de l'enzyme de conversion (Périndopril n = 3, Ramipril n = 2, Enalapril n = 1, Lisinopril n = 1, Fosinopril n = 1) et une insuffisance cardiaque légère à modérée de classe 1 ou 2 selon la classification NYHA. Ces patients ont reçu de l'ivabradine à la dose de 7,5 mg 2 fois par jour pour 7 d'entre eux et de 5 mg 2 fois par jour pour 1 d'entre eux. Après 2 mois de traitement, durée reconnue comme suffisante pour atteindre l'effet maximum des deux traitements, les pressions artérielles systoliques et diastoliques moyennes allongées ont baissé de 7,5 mmHg et 7,3 mmHg respectivement.
De plus si on considère les patients qui à l'inclusion dans cette étude avaient une pression artérielle qui n'était pas bien contrôlée sous inhibiteur de l'enzyme de conversion, c'est-à-dire ceux qui avaient encore une pression artérielle systolique ≥ 140 mmHg et/ou une pression artérielle diastolique ≥ 90 mmHg, la baisse de la pression artérielle dans ce groupe a été de 10 mmHg pour la pression artérielle systolique et de 8 mmHg pour la pression artérielle diastolique.

**Tableau 1 :**

| ***Evolution de la pression artérielle couchée à l'inclusion et après 2 mois de traitement avec un IEC et ivabradine*** | | |
|---|---|---|
| | Inclusion | 2 mois |
| PAS (mmHg) | 149,5 | 141,8 |
| PAD (mmHg) | 81,7 | 74,4 |

| | | |
|---|---|---|
| PAS : Pression artérielle systolique couchée PAD : Pression artérielle diastolique couchée | | |

**Tableau 2 :**

| ***Evolution de la pression artérielle couchée à l'inclusion et après 2 mois de traitement chez les patients qui présentaient une pression artérielle non contrôlée à l'entrée dans l'essai.*** | | |
|---|---|---|
| | Inclusion | 2 mois |
| PAS (mmHg) | 159 | 149 |
| PAD (mmHg) | 85 | 77 |

| | | |
|---|---|---|
| PAS : Pression artérielle systolique couchée PAD : Pression artérielle diastolique couchée | | |

Il a donc été observé une baisse substantielle de la pression artérielle lorsque l'on a ajouté l'ivabradine à un inhiteur de l'enzyme de conversion. Cette baisse de pression artérielle est inattendue car avec l'ivabradine la baisse moyenne qui est objectivée dans les essais cliniques est en général de l'ordre de 1 à 2 mmHg pour la pression artérielle diastolique et plutôt une légère augmentation de la pression artérielle systolique de l'ordre de 1 mmHg. Cette baisse est importante car on sait qu'une baisse de 4 à 5 mmHg chez les patients hypertendus diminue de façon très importante (30%) la survenue d'accidents cardiaques et neurologiques.

### Etude clinique n°2 :

Une étude clinique complémentaire a été réalisée chez des patients présentant une hypertension artérielle ainsi qu'une insuffisance cardiaque sévère de classe 3 selon la classification NYHA correspondant à une cardiopathie entraînant une limitation marquée de l'activité physique sans gêne au repos. Les patients ont été traités sur une durée de 6 semaines, durée reconnue comme suffisante pour observer clairement l'effet des traitements. Ces patients ont reçu par administration per os le traitement suivant :
- de l'ivabradine à la dose de 2.5mg 2 fois par jour pendant 2 semaines ; puis
- pendant les 2 semaines suivantes, de l'ivabradine à la dose de 5mg 2 fois par jour à l'exception des patients présentant une intolérance au produit, telle qu'une bradycardie excessive ou une symptomatologie clinique associée à une bradycardie marquée ; puis
- pendant les 2 dernières semaines, de l'ivabradine à la dose de 7.5mg 2 fois par jour sauf pour les patients présentant une intolérance au produit, telle qu'une bradycardie excessive ou une symptomatologie clinique associée à une bradycardie marquée.

40 patients déjà traités par un inhibiteur de l'enzyme de conversion tel que périndopril, ramipril, enalapril, lisinopril ou fosinopril ont reçu le traitement décrit supra. Les pressions artérielles systoliques et diastoliques moyennes allongées ont respectivement baissé de 2.5mmHg et 3.8mmHg.

**Tableau 3 :**

| ***Evolution de la pression artérielle couchée à l'inclusion et après 6 semaines de traitement avec un IEC et ivabradine*** | | |
|---|---|---|
| | Inclusion | 6 semaines |
| PAS (mmHg) | 126.3 | 123.8 8 |
| PAD (mmHg) | 78.4 | 74.6 |

| | | |
|---|---|---|
| PAS : Pression artérielle systolique couchée PAD : Pression artérielle diastolique couchée | | |

En outre, 11 patients, qui à l'inclusion dans cette étude avaient une pression artérielle qui n'était pas bien contrôlée sous inhibiteur de l'enzyme de conversion, c'est-à-dire ceux qui avaient encore une pression artérielle systolique ≥ 140mmHg et/ou une pression artérielle diastolique ≥ 90mmHg, ont reçu le traitement décrit supra. La baisse de la pression artérielle observée dans ce groupe a été de 10.7mmHg pour la pression artérielle systolique et de 8.6mmHg pour la pression artérielle diastolique.

**Tableau 4 :**

| ***Evolution de la pression artérielle couchée à l'inclusion et après 6 semaines de traitement avec un IEC et ivabradine, chez des patients présentant une pression artérielle non contrôlée à l'inclusion*** | | |
|---|---|---|
| | Inclusion | 6 semaines |
| PAS (mmHg) | 142.1 | 131.4 |
| PAD (mmHg) | 87.4 | 78.8 |

| | | |
|---|---|---|
| PAS : Pression artérielle systolique couchée PAD : Pression artérielle diastolique couchée | | |

Un groupe de 6 patients déjà traités avec périndopril comme inhibiteur spécifique de l'enzyme de conversion a reçu le traitement décrit supra. Une baisse significative de la pression artérielle a été observée à l'issue de 6 semaines de traitement, soit une baisse de 17.5mmHg de la pression artérielle systolique et de 7.7mmHg de la pression artérielle diastolique. Conformément aux conclusions de l'étude clinique précédente, cette baisse de la pression artérielle systolique et diastolique est considérée comme très importante quand on sait qu'une baisse de 4 à 5mmHg chez les patients hypertendus diminue de façon très importante (30%) la survenue d'accidents cardiaques et neurologiques.

**Tableau 5 :**

| ***Evolution de la pression artérielle couchée à l'inclusion et après 6 semaines de traitement avec périndopril et ivabradine*** | | |
|---|---|---|
| | Inclusion | 6 semaines |
| PAS (mmHg) | 127 | 109.5 |
| PAD (mmHg) | 77.5 | 69.8 |

| | | |
|---|---|---|
| PAS : Pression artérielle systolique couchée PAD : Pression artérielle diastolique couchée | | |

## Revendications

1. Association de l'inhibiteur du courant I_{f} sinusal ivabradine ou 3-{3r-[{[(7S) -3,4-diméthoxybicyclo[4.2.0]octa-1,3,5-trién-7-yl]méthyl}(méthyl)amino]propyl}-7,8-di-méthoxy-1,3,4,5-tétrahydro-2*H*-3-benzazépin-2-one, ou un de ses hydrates, formes cristallines ou sels d'addition à un acide pharmaceutiquement acceptable et d'un agent inhibiteur de l'enzyme de conversion de l'angiotensine.

2. Association selon la revendication 1 dans laquelle l'inhibiteur du courant I_{f} sinusal est le chlorhydrate d'ivabradine, l'ivabradine sous forme d'un de ses hydrates ou l'ivabradine sous forme d'une de ses formes cristallines.

3. Association selon la revendication 1 dans laquelle l'agent inhibiteur de l'enzyme de conversion de l'angiotensine est le périndopril, ou un de ses hydrates, formes cristallines ou sels d'addition à une base pharmaceutiquement acceptable.

4. Association selon la revendication 1 dans laquelle l'agent inhibiteur de l'enzyme de conversion de l'angiotensine est le périndopril, sous forme de sels de *ter*t-butylamine, eu sous forme de sel d'arginine, sous forme d'un de ses hydrates ou sous forme d'une de ses formes cristallines.

5. Association selon la revendication 1 dans laquelle l'inhibiteur du courant I_{f} sinusal est le chlorhydrate d'ivabradine, l'ivabradine sous forme d'un de ses hydrates ou l'ivabradine sous forme d'une de ses formes cristallines et l'agent inhibiteur de l'enzyme de conversion de l'angiotensine est le périndopril, sous forme de sels de *tert*-butylamine, sous forme de sel d'arginine, sous forme d'un de ses hydrates ou sous forme d'une de ses formes cristallines.

6. Compositions pharmaceutiques contenant comme principe actif l'inhibiteur du courant I_{f} sinusal ivabradine ou 3-{3-[{[(7S) -3,4-diméihoxybicyclo[4.2.0]octa-1,3,5-trién-7-yl]méthyl}(méthyl)amino]propyl}-7,8-diméthoxy-1,3,4,5-tétrahydro-2*H*-3-benzazépin-2-one, ou un de ses hydrates, formes cristallines ou sels d'addition à un acide pharmaceutiquement acceptable en association avec un agent inhibiteur de l'enzyme de conversion selon l'une des revendications 1 à 5, seuls ou en combinaison avec un ou plusieurs excipients pharmaceutiquement acceptables.

7. Compositions pharmaceutiques selon la revendication 6 contenant comme principe actif le chlorhydrate d'ivabradine, l'ivabradine sous forme d'un de ses hydrates ou l'ivabradine sous forme d'une de ses formes cristallines et le périndopril, sous forme de sels de *tert*-butylamine, sous forme de sel d' arginine, sous forme d'un de ses hydrates ou sous forme d'une de ses formes cristallines.

8. Compositions pharmaceutiques selon l'une des revendication 6 ou 7 utiles pour la fabrication d'un médicament pour le traitement de l'hypertension artérielle.

9. Utilisation d'une association selon l'une des revendications 1 à 5 pour l'obtention de compositions pharmaceutiques destinées au traitement de l'hypertension artérielle.

## Claims

1. Association of the sinus node I_{f} current inhibitor ivabradine, or 3-{3-[{[(7S)-3,4-dimethoxybicyclo[4.2.0]octa-l,3,5-trien-7-yl]methyl}(methyl)amino]propyl}-7,8-dimethoxy-1,3,4,5-tetrahydro-2*H*-3-benzazepin-2-one, or one of its hydrates, crystalline forms or addition salts with a pharmaceutically acceptable acid and an agent that inhibits angiotensin-converting enzyme.

2. Association according to claim 1, wherein the sinus node I_{f} current inhibitor is ivabradine hydrochloride, ivabradine in the form of one of its hydrates or ivabradine in the form of one of its crystalline forms.

3. Association according to claim 1, wherein the agent that inhibits angiotensin-converting enzyme is perindopril, or one of its hydrates, crystalline forms or addition salts with a pharmaceutically acceptable base.

4. Association according to claim 1, wherein the agent that inhibits angiotensin-converting enzyme is perindopril in *tert*-butylamine salt form, in arginine salt form, in the form of one of its hydrates or in the form of one of its crystalline forms.

5. Association according to claims 1, wherein the sinus node I_{f} current inhibitor is ivabradine hydrochloride, ivabradine in the form of one of its hydrates or ivabradine in the form of one of its crystalline forms, and the agent that inhibits angiotensin-converting enzyme is perindopril in *tert*-butylamine salt form, in arginine salt form, in the form of one of its hydrates or in the form of one of its crystalline forms.

6. Pharmaceutical compositions comprising as active ingredient the sinus node I_{f} current inhibitor ivabradine, or 3-{3-[{[(7S)-3,4-dimethoxybicyclo[4.2.0]octa-1,3,5-trien-7-yl]methyl}(methyl)amino]propyl}-7,8-dimethoxy-1,3,4,5-tetrahydro-2*H*-3-benzazepin-2-one, or one of its hydrates, crystalline forms or addition salts with a pharmaceutically acceptable acid, in association with an agent that inhibits angiotensin-converting enzyme, in accordance with one of claims 1 to 5, on their own or in combination with one or more pharmaceutically acceptable excipients.

7. Pharmaceutical compositions according to claim 6, comprising as active ingredient ivabradine hydrochloride, ivabradine in the form of one of its hydrates or ivabradine in the form of one of its crystalline forms, and perindopril in *tert*-butylamine salt form, in arginine salt form, in the form of one of its hydrates or in the form of one of its crystalline forms.

8. Pharmaceutical compositions according to one of claims 6 or 7 for use in the manufacture of a medicament for the treatment of arterial hypertension.

9. Use of an association according to one of claims 1 to 5 in obtaining pharmaceutical compositions intended for the treatment of arterial hypertension.

## Patentansprüche

1. Kombination aus dem Inhibitor des Sinusstroms I_{f} Ivabradin oder 3-{3-[{[(7S)-3,4-Dimethoxybicyclo[4.2.0]octa-1,3,5-trien-7-yl]-methyl}(methyl)-amino]-propyl}-7,8-di-methoxy-1,3,4,5-tetrahydro-2*H*-3-benzazepin-2-on oder eines seiner Hydrate, Kristallformen oder Additionssalze mit einer pharmazeutisch annehmbaren Säure und einem Inhibitor des Angiotensin umwandelnden Enzyms.

2. Kombination nach Anspruch 1, worin der Inhibitor des Sinusstroms I_{f} Ivabradin-Hydrochlorid oder Ivabradin in Form eines seiner Hydrate oder Ivabradin in Form einer seiner Kristallformen ist.

3. Kombination nach Anspruch 1, worin der Inhibitor des Angiotensin umwandelnden Enzyms Perindopril oder eines seiner Hydrate, Kristallformen oder Additionssalze mit einer pharmazeutisch annehmbaren Base ist.

4. Kombination nach Anspruch 1, worin der Inhibitor des Angiotensin umwandelnden Enzyms Perindorpil in Form des *tert*.-Butylaminsalzes, in Form des Argininsalzes, in Form seiner Hydrate oder in Form einer seiner Kristallformen ist.

5. Kombination nach Anspruch 1, worin der Inhibitor des Sinusstroms I_{f} Ivabradin-Hydrochlorid, Ivabradin in Form eines seiner Hydrate oder Ivabradin in Form einer seiner Kristallformen ist und der Inhibitor des Angiotensin umwandelnden Enzyms Perindopril in Form seines *tert*.-Butylaminsalzes, in Form seines Argininsalzes, in Form eines seiner Hydrate oder in Form einer seiner Kristallformen ist.

6. Pharmazeutische Zubereitungen enthaltend als Wirkstoff den Inhibitor des Sinusstroms I_{f} Ivabradin oder 3-{3-[{[(7S)-3,4-Dimethoxybicyclo[4.2.0]octa-1,3,5-trien-7-yl]-methyl}(methyl)-amino]-propyl}-7,8-di-methoxy-1,3,4,5-tetrahydro-2*H*-3-benzazepin-2-on oder eines seiner Hydrate, Kristallformen oder Additionssalze mit einer pharmazeutisch annehmbaren Säure in Kombination mit einem Inhibitor des Angiotensin umwandelnden Enzyms gemäß einem der Ansprüche 1 bis 5, allein oder in Kombination mit einem oder mehreren pharmazeutisch annehmbaren Hilfsstoffen.

7. Pharmazeutische Zubereitungen nach Anspruch 6, enthaltend als Wirkstoff Ivabradin-Hydrochlorid, Ivabradin in Form eines seiner Hydrate oder Ivabradin in Form einer seiner Kristallformen und Perindopril in Form des *tert*.-Butylaminsalzes, in Form des Argininsalzes, in Form eines seiner Hydrate oder in Form einer seiner Kristallformen.

8. Pharmazeutische Zubereitungen nach einem der Ansprüche 6 oder 7, nützlich für die Herstellung eines Arzneimittels zur Behandlung der arteriellen Hypertension.

9. Verwendung einer Kombination nach einem der Ansprüche 1 bis 5 zur Herstellung von pharmazeutischen Zubereitungen, die bestimmt sind zur Behandlung von arterieller Hypertension.
